(19) Europäisches Patentamt

European Patent Office

Office européen des brevets

(11) **EP 0 790 498 B1**

(12) **EUROPÄISCHE PATENTSCHRIFT**

(45) Veröffentlichungstag und Bekanntmachung des Hinweises auf die Patenterteilung:
**03.05.2006 Patentblatt 2006/18**

(51) Int Cl.:
*G01N 27/327* (2006.01) *C12Q 1/00* (2006.01)

(21) Anmeldenummer: **97101600.1**

(22) Anmeldetag: **03.02.1997**

(54) **Elektrochemische Sensoren mit verbesserter Selektivität und erhöhter Empfindlichkeit**

Electrochemical sensors with improved selectivity and increased sensitivity

Capteurs électrochimiques à sélectivité améliorée et à sensibilité augmentée

(84) Benannte Vertragsstaaten:
**AT BE CH DE DK ES FI FR GB IT LI**

(30) Priorität: **15.02.1996 DE 19605583**

(43) Veröffentlichungstag der Anmeldung:
**20.08.1997 Patentblatt 1997/34**

(73) Patentinhaber: **Bayer HealthCare AG
51368 Leverkusen (DE)**

(72) Erfinder:
• **Hildenbrand, Karlheinz, Dr.
47802 Krefeld (DE)**
• **Siegmund, Hans-Ulrich, Dr.
51381 Leverkusen (DE)**

(56) Entgegenhaltungen:
**EP-A- 0 276 782      EP-A- 0 471 986
EP-A- 0 546 536      WO-A-94/27140
DE-A- 2 021 285**

**Beschreibung**

Die vorliegende Erfindung beschreibt amperometrische Sensoren, vorzugsweise elektrochemische Biosensoren. Es wird auch ein Verfahren zur Herstellung von amperometrischen Biosensoren für den Bereich der Diagnose von Körperflüssigkeiten beschrieben.

Die Verwendung von amperometrischen Biosensoren, insbesondere im Bereich der Blutzuckerdiagnose ist seit einigen Jahren Stand der Technik.

Beschrieben sind solche Produkte beispielsweise in US-Patent 45 45 382, in EP 127 958, EP 351 891 und EP Appl. 0 47 1 986. Die entsprechenden Testsysteme sind unter den Produktnamen MediSense®, ExacTex® und Glucocard® kommerziell erhältlich. Sie erlauben die einfache Blutzuckerdiagnose unter home user-Bedingungen.

Eine besondere Bedeutung haben die amperometrischen Biosensoren mit Glucoseoxidase als Rezeptorkomponente erlangt. Wie in Anal. Chem. 1990, 62 1111 bis 1117 detailliert beschrieben, wird durch die Umsetzung von Glucose mit Glucoseoxidase eine der Zuckerkonzentration proportionale Wasserstoffperoxidmenge erzeugt.

Da die anodische Oxidation $H_2O_2 \rightarrow O_2 + 2H^+ + 2e^-$ jedoch eine relativ hohe Zellspannung (ca: 600 mV) erfordert, kann es bei der Vollblutanalyse zu unerwünschten Interferenzproblemen kommen. Bei der obengenannten Spannung werden nämlich bestimmte Blutkomponenten, wie Ascorbinsäure, ebenfalls umgesetzt, wobei es dann zu falsch positiven Ergebnissen kommt.

Im Hinblick auf Selektivitätsverbesserung bei amperometrischen Sensoren wurde daher das Konzept der Mediatoren entwickelt. Häufig eingesetzte Mediatoren bei den Biosensoren der sogenannten 2. Generation sind beispielsweise Ferrocene oder Kaliumhexacyanoferrat $K_3Fe\,(CN)_6$. Die amperometrische Blutzuckerbestimmung verläuft in diesem Falle nach dem folgenden Reaktionsschema:

(1) Glucose + GO (FAD) $\rightarrow$ Gluconolacton + GO (FADH$_2$)
(2) GO (FADH$_2$) + Fe(III)(CN)$_6{}^{3-}$ $\rightarrow$ Fe(II)(CN)$_6{}^{4-}$ + GO (FAD) + H$^+$
(3) Fe(II)(CN)$_6{}^{4-}$ $\rightarrow$ Fe(III)(CN)$_6{}^{3-}$ + e$^-$

Die amperometrische Blutzuckerbestimmung beschränkt sich meßtechnisch somit auf die in (3) beschriebene anodische Oxidation, die bei einem Potential von +360 mV abläuft. Derartige Mediator modifizierte Biosensoren weisen damit eine erhöhte Selektivität auf.

Im Hinblick auf reproduzierbare Ergebnisse muß die $O_2$-gesteuerte Nebenreaktion GO (FADH$_2$) + $O_2$ $\rightarrow$ GO (FAD) + $H_2O_2$ möglichst vollständig verhindert werden.

Der Aufbau eines geeigneten Testmittels enthält neben den erforderlichen Nachweisreagenzien, beispielsweise Glucoseoxidase und Kaliumhexacyanoferrat, noch mindestens zwei Elektroden (Arbeitselektrode und Referenzelektrode), die über eine Elektrolytbrücke in Kontakt stehen müssen.

Als Elektrodenmaterialien kommen dem Stand der Technik entsprechend Edelmetalle, wie Palladium, Platin, Gold, Silber und Kupfer oder Graphit in Frage, wobei Anode (Arbeitselektrode) und Kathode (Referenzelektrode) aus verschiedenen Materialien oder aus demselben Material hergestellt werden können und gleich große oder verschieden große Oberflächen besitzen können.

Der Testablauf bei den kommerziell verfügbaren Systemen beschränkt sich für den Patienten auf die Aufgabe der Probenflüssigkeit (Vollblut), wobei der Analysenwert digital innerhalb von wenigstens einer Minute angezeigt wird.

Der tatsächliche Reaktionsablauf, bei dem der Analyt (Glucose) oxidiert und der Mediator reduziert wird, ist jedoch meßtechnisch so geregelt, daß die folgenden Schritte eingehalten werden:

a) Blutaufgabe und Reaktionsablauf entsprechend (1) bis (2).

b) Nach Einhalten einer gewissen Reaktionszeit von ca. 5 bis 30 sec wird eine konstante Spannung von ca. 400 mV angelegt, wobei die in (3) beschriebene anodische Oxidation stattfindet.

c) Nach einer kurzen Verzögerungszeit wird der Strom gemessen.

Die analytische Auswertung erfolgt im Bereich der Diffusionsgrenzströme, wobei nach der sogenannten Cottrell-Gleichung

$$i\,(t) = \frac{n \circ F \circ A \sqrt{D} \circ C}{\sqrt{\pi}\,\sqrt{t}} \qquad \text{Gleichung (A)}$$

i = Strom

n = Zahl der Elektronen bei der Elektrodenreaktion

F = Faraday-Konstante

D = Diffusionskoeffizient

C = Konzentration des Analyten

A = Fläche der Arbeitselektrode

D = Dicke der Diffusionsgrenzschicht a.d. Arbeitselektrode

t = Zeit

Um diese Bedingungen zu erfüllen, ist es erforderlich, daß die oxidierte Form des Redox-Mediators ($K_3Fe(CN)_6$) an der Gegenelektrode die Konzentration der reduzierten Mediatorform ($K_4Fe(CN)_6$) an der Arbeitselektrode deutlich überbietet.

Testmittel, die eine separate Auftragung des Mediatorsystems beispielsweise Kaliumhexacyanoferrat ($K_3Fe(CN)_6$) auf die Gegenelektrode sowie des enzymatischen Rezeptors (GO) auf die Arbeitselektrode ermöglichen - gegebenenfalls durch Immobilisierung fixiert - sollten dementsprechend von Vorteil sein.

Testsysteme mit separierten Reagenzzonen können auch im Hinblick auf Langzeitstabilität des enzymatischen Reagenzsystems von Vorteil sein.

In einer Reihe von verschiedenen Publikationen sind weitere erstrebenswerte Eigenschaftsmerkmale für elektrochemische Biosensoren aufgeführt, die zu einer Optimierung des Gesamtsystems beitragen können.

Einige wichtige seien im folgenden aufgeführt:

**- Weitere Erhöhung der Selektivität**

Europ. Pat. Spez. 0.276 782 beschreibt Enzymelektroden mit durch Glutardialdehyd vernetzten Albuminschichten, die aufgrund ihrer Permeabilität die Arbeitselektrode vor elektroaktiven Störkomponenten, v.a. vor höhermolekularen Proteinen schützen.

Die Verwendung von synthetischen Membranen für den Ausschluß der roten Blutzellen bei elektrochemischen Zellen ist in Europ. Pat. Appl. 0 289 265 beschrieben.

In WO 94/27140 sind elektrochemische Sensoren mit Erythrocyten-Exklusionsmembranen beschrieben, die mobile Erythrocyten Agglutinationsmittel enthalten.

In Europ. Pat. Appl. 0 546 536 ist ein System mit einer zweigeteilten Arbeitselektrode bestehend aus einem enzymfreien und einem enzymhaltigen Feld beschrieben, wobei ersteres die nicht enzymatisch umsetzbaren oxidierbaren Störkomponenten, wie Ascorbinsäure, erfaßt. Der korrigierte tatsächliche Blutzuckerwert wird dann kalkulatorisch aus den Einzelpotentialmessungen ermittelt.

Nankai et al. beschreiben in WO 86/07 642 ein Dreielektrodensystem, das neben Arbeits- und Referenzelektrode noch eine Bezugselektrode enthält, die die Abhängigkeit der Zellspannung vom Zellstrom ausgleicht.

**- Steigerung der Sensorempfindlichkeit**

Die Erhöhung der Empfindlichkeit durch Vergrößern der Elektrodenoberflächen entsprechend Gleichung (A) ist in EP 0 385 964 beschrieben.

**- Verbesserte Handhabbarkeit**

Nankai et al. beschreiben in Eur. Appl. 0 471 986 die Herstellung eines amperometrischen Blutzuckertestsystems mit Einmalsensoren, wobei sich dieses System durch eine besonders gute Handhabbarkeit auszeichnet. Der im amperometrischen Auswertegerät steckende Einmalsensor wird mit der Sensorspitze an den zu analysierenden Bluttropfen getippt. Über eine Mikrokapillare (kapillares Fließsystem) wird Vollblut in den Sensorarbeitsraum (Arbeits- bzw. Referenzelektrode plus Nachweisreagenzien) transportiert. Hierbei lösen sich die Nachweisreagenzien ($GO/K_3Fe(CN)_6$) in der zu analysierenden Flüssigkeit (Blut) auf und es läuft die bereits zitierte Nachweisreaktion ab. Sind beide Elektroden mit Blut benetzt - Voraussetzung für störungsfreie Funktionsfähigkeit - so erfolgt aufgrund des verringerten Widerstandswertes automatisch der Start des Auswertegerätes. Das Gerät kann deshalb ohne jeglichen Bedienungsknopf gehandhabt werden. Im Hinblick auf schmerzarme Blutgewinnung wird die erforderliche Blutmenge möglichst gering gehalten und daher das Volumen des Mikrokapillarsystems auf ca. 5 $\mu$l beschränkt. Aus dem durch die Mikrokapillare begrenzten Reaktionsraum führen Leiterbahnen über den verlängerten Sensorteil zu den Steckkontakten, eine Verunreinigung von wichtigen Funktionsteilen im Auswertegerät ist somit ausgeschlossen.

Zur Herstellung der zitierten Blutglucose-Biosensoren wird üblicherweise das Verfahren der Siebdrucktechnologie eingesetzt.

Für den Druckprozeß des Elektrodenteils (Transducer) werden kommerziell erhältliche Siebdruckpasten, beispielsweise auf Graphit- oder Silberbasis (Fa. Acheson) eingesetzt, die auf Trägermaterialien, wie Keramik oder Kunststoff-Folien aufgedruckt werden. Hierbei sind mehrere aufeinanderfolgende Druck- und Trocknungsschritte (Leiterbahnen, Arbeitselektroden, Referenzelektroden, Dielektrikumschichten) erforderlich.

Die Siebdruckpasten, die hinsichtlich Verarbeitbarkeit eine Reihe verschiedener Additive, wie Entschäumer, Thixotropiemittel und Detergentien, enthalten, weisen hinsichtlich Reproduzierbarkeit oft erhebliche Mängel auf.

Häufig müssen die siebgedruckten Elektrodenoberflächen noch durch Plasmabehandlung aktiviert werden. Wegen des hohen, relativ hydrophoben Bindemittelanteils sind nämlich deren Oberflächen eher hydrophob, schlecht benetzt und in der Leitfähigkeit gegenüber dem reinen Leitermaterial, beispielsweise Graphit oder Silber stark reduziert.

Weitere Nachteile der Plasmabehandlung, wie Alterung oder Erzeugung unerwünschter redoxaktiver Oberflächengruppen müssen einkalkuliert werden. Im Anschluß an die Herstellung des Elektrodenteils erfolgt die Auftragung der Nachweisreagenz-Rezeptur, beispielsweise Glucoseoxidase (GO) und Kaliumhexacyanoferrat im Falle des Blutzuckernachweises. Hierbei muß jede einzelne Sensor-Arbeitsfläche einzeln dotiert werden, wobei entweder ebenfalls das Verfahren der Siebdrucktechnologie oder das aufwendige Verfahren der Mikropipettierung angewandt wird.

In einem dritten Verfahrensprozeß wird schließlich das Mikrokapillarsystem durch Verkleben entsprechend vorgeformter Folien, die gegebenenfalls im Hinblick auf gute Benetzbarkeit mit hydrophilen Schichten versehen werden müssen, aufgebracht.

Insgesamt gesehen handelt es sich also um einen relativ komplizierten Herstellungsprozeß.

Es wurde nun überraschenderweise ein Verfahren zur Herstellung elektrochemischer Sensoren gefunden, das hinsichtlich Herstellung deutlich vereinfacht und hinsichtlich Reproduzierbarkeit sicherer ist.

Das erfindungsgemäße Verfahren erlaubt es insbesondere, die im Text beschriebenen angestrebten Eigenschaften, die zu einer Produktverbesserung führen sollten, in einem System zu vereinigen. Beim bisherigen Stand sind diese kombinierten und integralen Eigenschaftsprofile noch nicht realisiert worden.

So ist eine Erhöhung der Sensitivität (Empfindlichkeit) in einfacher Weise durch eine Vergrößerung der Reagenzmatrix-Fläche möglich, ohne daß die benötigten Probenvolumina (z.B. Bluttropfen) - wie beim konventionellen System - deutlich erhöht werden müssen.

Eine Selektivitätssteigerung ist durch die Integration von porösen Trenn-Schichten möglich. Wie im folgenden näher beschrieben, können nämlich in verschiedenen Sensorschichten, beispielsweise poröse Referenzelektrode, Membranen als Reagenzmatrix und gegebenenfalls über Membranbeschichtung der Arbeitselektrode, selektivitätssteigernde Trennprozesse integriert werden.

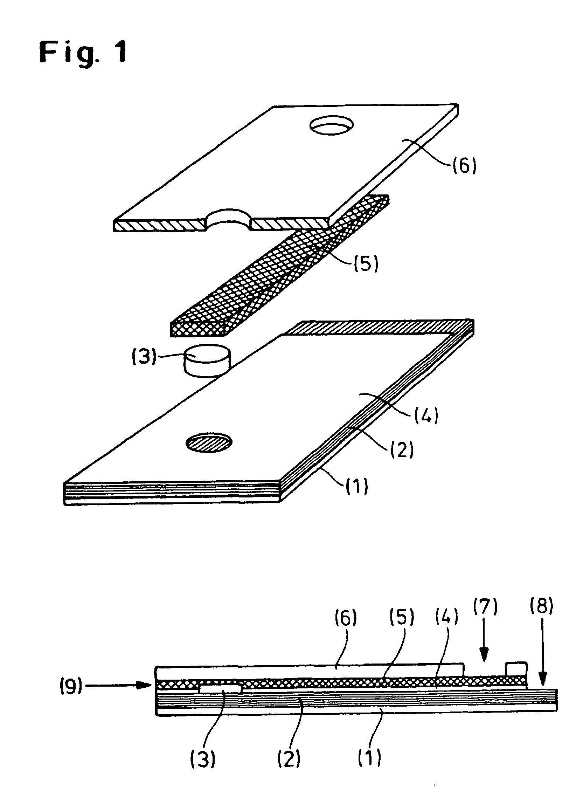

Zur Verdeutlichung der erfindungsgemäßen Sensorsysteme soll die Fig. 1 dienen:

Auf eine Graphit-Folie (2), die auf eine Basisfolie (1) fixiert ist, wird eine Reagenzmatrix (Membran) (3) mit einer Fläche im Bereich von einigen mm$^2$ aufgebracht. Darüber wird mittels eines durchlochten Doppelklebebandes (4) ein Streifen eines Graphitvlieses (5) befestigt. Als obere Abdeckung wird schließlich eine durchlochte Folie (6) aufgeklebt. Das erforderliche Probevolumen kann durch Integration einer Flüssigkeitsstopzone im Graphitvlies, beispielsweise hinter der Reagenzmatrix (Membran) (3), definiert werden.

Die Kontaktierung des Potentiostaten erfolgt an (7) zur Graphitvliesschicht (Referenzelektrode, Kathode) sowie an (8) zur Graphitfolie (Arbeitselektrode, Anode). Die Probenaufgabe kann über die Stirnkante (9) des Graphitvlieses erfolgen, wobei es - wie in den Beispielen beschrieben - zu einem Flüssigkeitstransport in Richtung der Reagenzmatrix kommt.

Im folgenden sind die für die einzelnen Funktionsschichten verwendeten bzw. möglichen Komponenten näher beschrieben:

**a) Arbeitselektrode**

Vorzugsweise eingesetzt wurden Graphit-Folien, die unter dem Markennamen Sigraflex® von der Fa. SGL CarbonGroup erhältlich sind.

Die für diesen Einsatzzweck wichtigen Kenngrößen sind:

Spezifischer elektrischer Widerstand: 8 bis 10 $\Omega\mu$m parallel zur Schicht
600 bis 650 $\Omega\mu$m senkrecht zur Schicht
Schichtdicke: 0,25 bis 1,00 mm
Reinheit: >99,85 %

Im Hinblick auf Steigerung der Reaktionsselektivität kann wie später in den Beispielen beschrieben wird, die der Reagenzmatrix (3) zugewandte Graphit-Oberfläche mit einer integralen Membranschicht versehen sein, wobei es sich entweder um eine mikroporöse Porenmembran oder um eine porenfreie, quellende Membranschicht handeln kann.

Als Alternative zu Graphit-Folien können andere bekannte Elektroden-Materialien, wie Gold, Silber oder Platin eingesetzt werden.

Eine Plasmabehandlung zur Verbesserung der Benetzbarkeit bzw. zur Erhöhung der Leitfähigkeit ist nicht erforderlich.

**b) (Permeable) Trägermatrix**

Permeable Flächengebilde, die hierzu in Frage kommen, können ausgewählt sein aus der Gruppe der Polymervliese, beispielsweise aus Polyester oder Polyvinylalkohol, Polymergewebe, beispielsweise aus Polyamid oder Polyester oder vorzugsweise aus der Gruppe der Polymermembranen.

Bevorzugte Polymermembranen sind solche, die der Gruppe der Mikrofiltration zugeordnet werden und im Porenbereich von ca. 0,1 bis 10 $\mu$m, besonders bevorzugt im Bereich von 0,3 bis 5 $\mu$m liegen und sich durch gute Benetzbarkeit auszeichnen. Beispiele hierfür sind Polyamidmembranen (Biodyne®) der Fa. PALL, hydrophilisierte Polyvinylfluoridmembranen (Durapore®) der Fa. Millipore, hydrophilisierte Polysulfonmembranen (Supor®) der. Fa. Gelmann oder Polymerblendmembranen wie sie in US 5 124 128 beschrieben sind.

Die eingesetzten Membrantypen können freitragend oder trägergestützt sein, wobei das Trägermaterial aus Polymervlies oder Polymergewebe bestehen kann und mittig oder einseitig in die Membranschicht integriert sein kann. Vom strukturellen Aufbau her können die eingesetzten Membranen asymmetrisch oder symmetrisch sein.

Ein besonderer Vorteil der erfindungsgemäßen Sensoren beruht auf dem dualen Charakter dieser speziellen Reagenzmatrizen, die sowohl Reagenz-Trägerfunktionen als auch Separationsfunktionen ausüben.

Die Auswahl der geeignetsten permeablen Trägermatrix richtet sich nach dem speziellen Anwendungsfall. So sind beispielsweise für den Blutzuckertest solche Membranen besonders gut geeignet, die Plasma gut permeieren lassen, die roten Blutzellen jedoch zurückhalten.

Es ist auch möglich, solche Trägermatrixsysteme einzusetzen, die eine Immobilisierung der Nachweis-Reagenzien, beispielsweise Glucoseoxidase ermöglichen. Denkbare Ausführungsformen wären dann beispielsweise kontinuierlich arbeitende oder wiederverwendbare Biosensoren.

Im Hinblick auf mehrstufige Nachweisreaktionen, beispielsweise Cholesterintest, können auch mehrere Trägermatrixsysteme übereinanderliegend kombiniert werden. Es ergeben sich dann mannigfache Möglichkeiten hinsichtlich Einarbeitung der Nachweisreagenzien sowie zur Abtrennung von unerwünschten Störkomponenten.

Wie im folgenden am Beispiel des Blutzuckertests etwas detaillierter beschrieben, bestehen bereits bei einschichtigen Matrix-Varianten im Vergleich zu Siebdruck oder Micropipetting verschiedenartige Reagenzien-Einarbeitungsmöglichkeiten, die beispielsweise hinsichtlich Reaktionsperformance oder Langzeitstabilität von Interesse sind: Die Trägermatrix kann im einfachsten Falle über konventionelle Tränkprozeduren, beispielsweise mit Glucoseoxidase und Kaliumhexacyanoferrat imprägniert werden.

Es ist auch möglich, eine pastöse Reagenzzubereitung auf eine oder beide Seiten der Matrix zu beschichten, wobei Tränk- und Beschichtungsprozeduren auch kombiniert werden können.

So wird beispielsweise wie in den Beispielen detailliert beschrieben, in einer bevorzugten Prozedur für den amperometrischen Blutzuckertest die Trägermatrix mit Kaliumhexacyanoferrat imprägniert, während eine pastöse Glucoseoxidase-Rezeptur auf die der Arbeitselektrode zugewandten Seite aufgetragen wird.

Bei allen Formen der Reagenzieneinarbeitung ist jedoch evident, daß im Vergleich zu Verfahren wie Siebdruck oder Micropipetting keine individuelle Sensordotierung sondern konventionelle in der Teststreifendiagnostik etablierte Verfahren eingesetzt werden können, woraus erhebliche produktionstechnische . Vorteile resultieren.

Die für den individuellen Sensor eingesetzten Reagenzmatrix-Flächen können ebenfalls in einem relativ breiten Rahmen variiert werden. So können beispielsweise für weniger sensitive oder im niedrigeren Konzentrationsbereich liegende Analyten mit Hilfe größerer Reagenz-Matrixflächen entsprechend Gleichung (A) noch gut zu interpretierende dose response-Signale erzeugt werden, ohne daß unverhältnismäßig große Probevolumina (beispielsweise Blut) benötigt werden.

Wegen der Möglichkeit, über die Reagenzmatrixfläche die Empfindlichkeit steigern zu können, sind die erfindungsgemäßen Sensoren insbesondere auch für immunchemische Nachweissysteme von großem Interesse.

Praktikable Matrixflächen liegen im Bereich von einigen mm$^2$. Wie in den Beispielen beschrieben, wurden zur Evaluierung des Blutzuckertests kreisförmige Matrix-Scheiben mit einem Durchmesser von 3 mm, was einer Fläche von etwa 7 mm$^2$ entspricht, eingesetzt.

Für die Funktion der damit hergestellten Biosensoren waren überraschenderweise Probevolumina im Bereich von ca. 2 $\mu$l ausreichend, wobei im Vergleich zu den bisher bekannten Blutzuckerbiosensoren deutlich höhere Stromausbeuten (ca. 8 fach erhöhte Werte) erzielt werden konnten.

Konventionelle Biosensoren beinhalten Arbeitselektrodenflächen von ca. 1 bis 2 mm$^2$, benötigen Probenvoluminamengen (Blut) von wenigstens 5 $\mu$l und erzielen eine Stromausbeute im Bereich von ca. 0,1 bis 20 $\mu$A.

Sensorsysteme, die mit minimalen Probevolumina arbeiten können, sind insbesondere im Rahmen der sogenannten "minimal invasive" Konzeote (PCT WO 95/10223) von Interesse, wobei Werte von 2 $\mu$l oder weniger angestrebt

werden.

### c) Poröse, leitfähige Referenzelektrode

Als bevorzugtes Material wurden, wie bereits erwähnt Graphitvliese eingesetzt, die beispielsweise unter dem Markennamen Sigrafil® SPC 7011 von der Fa. SGL Carbon Group zu beziehen sind.

Es handelt sich hierbei um schwarze, sehr reißfeste Vliese mit einem Flächengewicht von 30 g/m$^2$, einer Dicke von 0,5 mm, einem mittleren Faserdurchmesser von 7 $\mu$m und einem Bindemittelsystem aus vernetztem Polyvinylalkohol, dessen Anteil bei ca. 20 bis 24 Gew.% liegt.

Wie bereits angedeutet zeichnet sich dieses Material durch zwei besondere Eigenschaften aus, die für die Herstellung von elektronischen Biosensoren von besonderem Interesse sind. Es sind dies die Fähigkeiten zum sehr schnellen und schonenden Flüssigkeitstransport sowohl in vertikaler als auch in horizontaler Richtung sowie seine elektrische Leitfähigkeit, wobei der spezifische elektrische Widerstand im Bereich von ca. 10 $\Omega\mu$m liegt.

Diese Graphitvlies-Schicht übt somit gleichzeitig die Funktion des kapillaren Flüssigkeitstransports als auch der Referenzelektrode aus.

Solche Graphitvliesschichten in Kombination mit Agglutinationsmitteln, wie Lectinen können auch in hervorragender Weise eine Blut/Plasmaseparation bewirken.

Derartige Separationsprozesse sind bei der Analyse von Blutproben im Hinblick auf Reduzierung des Hämatokriteinflusses von großem Interesse.

Als Alternative zu den erwähnten Graphitvliesen können auch andere elektrisch leitende poröse Flächengebilde, wie metallisierte Gewebe, Vliese oder Membranen eingesetzt werden, die zur Verbesserung der Benetzbarkeit mit oberflächenaktiven Substanzen behandelt werden können.

Als Beispiel für ein geeignetes elektrisch leitendes Gewebe sei die Type Metalen 120 bis 34 T der Fa. SEFAR angeführt.

Es handelt sich hierbei um ein vernickeltes multifiles Polyestergewebe.

Leitfähige Membranen sind beispielsweise von der Fa. Millipore auf Basis von reinem Silber zu erhalten.

Es ist auch möglich, für diesen Zweck der leitfähigen, porösen Referenzelektrode konventionelle Membranen, die nach einem der gängigen Verfahren metallisiert worden sind, einzusetzen.

### d) Kunststoff-Folien

Die Basisfolie (1) bzw. die obere Abdeckfolie (6) kann prinzipiell ohne größeres Auswahlverfahren aus dem breiten Sortiment der Kunststoff-Folien ausgewählt werden.

Im Hinblick auf die mechanische Stabilität des Biosensorstreifens sind jedoch Folien mit bestimmten Steifigkeiten und Schichtdicken bevorzugt.

Eingesetzt wurden beispielsweise Polyester-, Polycarbonat- und PVC-Folien im Dickenbereich von ca. 100 bis 300 $\mu$m, die teilweise transparent oder pigmentiert waren.

Im Hinblick auf einen verbesserten Flüssigkeitstransport kann es von Vorteil sein, wenn die Innenseite der Deckfolie eine hydrophile Substratschicht trägt. Derartig modifizierte Folien sind beispielsweise im Standardfoliensortiment der Firmen ICI oder Du-Pont enthalten.

Das Verkleben bzw. Laminieren der einzelnen Schichten kann, wie erwähnt, mit Hilfe von Klebebändern, Heißschmelzklebern oder einem der bekannten Schweißverfahren erfolgen.

### Beispiel

Es wurde ein amperometrisches Testmittel gemäß Abb. 1 aufgebaut:

| | | |
|---|---|---|
| (1) | Basisfolie | (Polycarbonatfolie, 250 $\mu$m dick) |
| (2) | Graphitfolie | (Sigraflex®, mit Doppelklebeband auf (1) befestigt) |
| (3) | Reagenzmembran | (Biodyne® Fa. PALL, imprägniert mit Glucose-oxidase und Kaliumhexacyanoferrat) |
| (4) | Doppelklebeband | |
| (5) | Graphitvlies | (Sigratex® SPC 7011) |
| (6) | Deckfolie | (Polycarbonatfolie, 250 $\mu$m dick) |

Die Kontaktierung zum Amperometer erfolgte am Graphitvlies bei (7) (Kathode, Referenzelektrode) bzw. an der Graphitfolie bei (8) (Anode, Arbeitselektrode)

Die Probenaufgabe (3 $\mu$l) erfolgte an der Stirnseite (9) des Graphitvlieses mit Hilfe einer Pipette. Dabei kam es zu einem kapillaren Flüssigkeitstransport in Richtung der Reagenzmatrix.

Präparation der Reagenzmatrix:

a) Imprägnierung mit Kaliumhexacyanoferrat

Eine Nylon-Membran der Fa. PALL (Biodyne, 0,45 μm) wurde mit einer 20 % Kaliumhexacyanoferratlösung imprägniert und getrocknet.

b) Einarbeitung von Glucoseoxidase

Mit Hilfe eines schnelldrehenden Rührers (Dissolver) wurde aus den folgenden Komponenten eine Glucose-oxidase enthaltende Gießlösung hergestellt:

| | |
|---|---|
| 4,42 g | Polyethylenoxid 300 000 (Union Carbide) |
| 84,08 g | Citratpuffer (0,01 molar pH = 6,5) |
| 0,58 g | Octanol-1 |
| 3,84 g | Aerosil® (hochdisperse Kieselsäure, Fa. Degussa) |
| 0,12 g | Tensid FC-170 C (Fa. 3M) |
| 7,00 g | Glucoseoxidase (150 μ/mg) |

Nach Entgasen wurde diese Gießlösung mit Hilfe eines Rakels (Naßauftrag 50 μm) auf die mit Kaliumhexa-cyanoferrat imprägnierte Nylon-Membran beschichtet und mit Warmluft getrocknet.

Mit Hilfe einer Revolverzange wurden aus der so hergestellten Reagenzmembran kreisrunde Scheiben mit einem Durchmesser von 3 mm ausgestanzt.

Nach Aufbau des in Abb. 1 dargestellten Formats wurden amperometrische Testserien bei 400 mV mit folgenden Testlösungen durchgeführt:

a) Wäßrige Generallösungen

0, 25, 50, 100, 200, 300, 400, 500 mg/dl Glucose

Es wurden Meßzeiten von 30 sec eingehalten, wobei entsprechend der Cottrell-Gleichung mit 1/t abfallende chronamperometrische Kurven erhalten wurden. Ent sprechend der zunehmenden Glucosekonzentration wurden Werte mit zunehmend erhöhten Stromdichten erhalten.

b) Vollblut

Es wurde frisches Vollblut mit einem Glucosewert von 104 mg/dl in Analogie zu a) appliziert. Es resultierte eine Meßkurve, die mit der 100 mg/dl Kurve aus a) weitgehend deckungsgleich war.

**2. Beispiel**

Herstellung membranbeschichteter Graphitfolien

a) Poröse Membranschicht

Mit Hilfe eines schnelldrehenden Rührers (Dissolver) wurden aus den folgenden Komponenten eine Gießlösung hergestellt:

| | | |
|---|---|---|
| Dralon L | (Bayer AG) | 50,0 g |
| Ultrason E | (BASF) | 50,0 g |
| Aerosil 200 | (Degussa) | 30,0 g |
| Pluriol P 600 | (BASF) | 90,0 g |
| N-Methylpyrrolidon | (NMP) | 484,0 g |

Nach Entgasen wurde diese Gießlösung mit Hilfe eines Rakels (Naßauftrag 150 μm) auf eine Graphit-Folie

EP 0 790 498 B1

(Sigraflex) beschichtet und in ein Wasserbad getaucht. Nach Trocknen und Imprägnieren mit Glucoseoxidase und Kaliumhexacyanoferrat wurde, wie in Beispiel 1 beschrieben, eine Membranscheibe von 3 mm Durchmesser ausgestanzt und das ebenfalls in Beispiel 1 beschriebene Format aufgebaut.

Bei der chronamperometrischen Auswertung wurden in Analogie zu Beispiel la mit steigenden Glucosekonzentrationen steigende Stromdichten erhalten.

b) Nichtporöse, quellende Membranschicht auf Graphitfolie

Mit Hilfe eines schnelldrehenden Rührers wurde aus dem folgenden Komponenten eine Gießlösung hergestellt:

| | | |
|---|---|---|
| 8,77 g | wäßrige Polyurethandispersion DLS | (Bayer AG) |
| 9,66 g | Polyethylenoxid 300 000 | (Union Carbide) |
| 0,06 g | Pluronic PE 6400 | (BASF) |
| 1,20 g | Citratpuffer (0,1 m, pH = 6,5) | |
| 0,34 g | Aerosil 200 | (Degussa) |
| 1,00 g | Glucoseoxidase (154 U/mg) | |

Nach Entgasen wurde diese Gießlösung mit Hilfe eines Rakels (Naßauftrag 100 $\mu$m) auf eine Graphitfolie (Sigraflex®) beschichtet, getrocknet und ausgestanzt (3 mm Kreisscheibe).

Die so beschichtete Arbeitselektrode wurde entsprechend (3) Abb. 1 eingesetzt. Bei der Auswertung mit wäßrigen Glucoselösungen wurden bei einer angelegten Spannung von 600 mV in Analogie zu Beispiel 1a mit zunehmenden Glucosekonzentrationen zunehmende Stromstärken gemessen.

Ergebnisse der Glucosesensoren mit membranbeschichteten Graphitfolien:

Während mit reinen, wäßrigen Glucoselösungen weitgehend analoge Ergebnisse wie in Beispiel 1 resultierten, wurden mit den membranmodifizierten Sensorsystemen im Hinblick auf Testlösungen, die interferierende Komponenten mitenthielten (Ascorbinsäure, Acetaminophen) verbesserte Ergebnisse erhalten.

Eine falsch positive Veränderung durch die Interferenzverbindung war praktisch vollständig eliminiert.

**Patentansprüche**

1. Amperometrischer Sensor, enthaltend eine Arbeitselektrode und eine Referenzelektrode sowie mindestens ein Nachweisreagenz,
   **dadurch gekennzeichnet,**
   **daß** die Arbeitselektrode und die Referenzelektrode durch ein elektrisch nicht leitendes permeables Flächengebilde, das ein Nachweisreagenz aufweist, voneinander getrennt sind, und
   **daß** die Referenzelektrode aus einem elektrisch leitenden porösen Flächengebilde besteht, das zum kapillaren Flüssigkeitstransport von einer Probenaufgabezone zu dem elektrisch nicht leitenden permeablen Flächengebilde dient.

2. Sensor gemäß Anspruch 1, **dadurch gekennzeichnet, daß** das elektrisch nicht leitende permeable Flächengebilde aus einem porösen Material, beispielsweise Vlies, Papier, Gewebe, Membran oder aus einer nichtporösen, quellfähigen Membranschicht besteht

3. Sensor gemäß den Ansprüchen 1 bis 2, **dadurch gekennzeichnet, daß** das elektrisch nicht leitende permeable Flächengebilde aus mehreren Schichten aufgebaut ist.

4. Sensor gemäß Anspruch 3, **dadurch gekennzeichnet, daß** die einzelnen Schichten unterschiedliche Nachweisreagenzien enthalten.

5. Sensor gemäß den Ansprüchen 1 bis 4, **dadurch gekennzeichnet, daß** das elektrisch nicht leitende permeable Flächengebilde eine Fläche im Bereich von von einigen mm$^2$, bevorzugt im Bereich von 7 mm$^2$ hat.

8

6.  Sensor gemäß den Ansprüchen 1 bis 5, **dadurch gekennzeichnet, daß** das elektrisch nicht leitende permeable Flächengebilde eine Porengröße im Bereich von 0,1 bis 10 $\mu$m, bevorzugt im Bereich von 0,3 bis 0,5 $\mu$m hat.

7.  Sensor gemäß den Ansprüchen 1 bis 6, **dadurch gekennzeichnet, daß** die Nachweisreagenzien mit dem elektrisch nicht leitenden permeablen Flächengebilde durch Tränkung oder Beschichtung in Verbindung gebracht werden.

8.  Sensor gemäß den Ansprüchen 1 bis 7, **dadurch gekennzeichnet, daß** die Nachweisreagenzien Glucoseoxidase und Kaliumhexacyanoferrat sind.

9.  Sensor gemäß den Ansprüchen 1 bis 8, **dadurch gekennzeichnet, daß** das elektrisch leitende poröse Flächengebilde aus metallisiertem Gewebe, Vlies oder Membran besteht.

10. Sensor gemäß den Ansprüchen 1 bis 9, **dadurch gekennzeichnet, daß** das elektrisch leitende poröse Flächengebilde
aus einem hydrophilen Graphitfasermaterial, vorzugsweise Graphitvlies, oder
aus einem nichtleitenden hydrophilen Vlies mit einer darüberliegenden leitfähigen Metall- oder Graphitfolie besteht.

11. Sensor gemäß den Ansprüchen 1 bis 10, **dadurch gekennzeichnet, daß** das elektrisch leitende poröse Flächengebilde weitere Reagenzien enthält.

**Claims**

1.  Amperometric sensor containing a working electrode and a reference electrode, and at least one detecting reagent, **characterized by**
the working electrode and the reference electrode being separated by an electrically non-conductive, permeable sheet with a detecting reagent
and
the reference electrode consisting of an electrically conductive, porous sheet, for capillary liquid transport from a sampling zone to the electrically non-conductive, permeable sheet.

2.  Sensor meeting the requirements of 1 **characterized by** the electrically non-conductive, permeable sheet consisting of a porous material, for example fleece, paper, fabric, membrane or a non-porous, swellable membrane layer.

3.  Sensor meeting the requirements of 1 to 2 **characterized by** the electrically non-conductive, permeable sheet consisting of several layers.

4.  Sensor meeting the requirements of 3 **characterized by** the individual layers containing different detecting reagents.

5.  Sensor meeting the requirements of 1 to 4 **characterized by** the electrically non-conductive, permeable sheet having an area in the region of a few mm$^2$, preferably in the region of 7 mm$^2$.

6.  Sensor meeting the requirements of 1 to 5 **characterized by** the electrically non-conductive, permeable sheet having a pore size in the range 0.1 to 10 $\mu$m, preferably in the range 0.3 to 0.5 $\mu$m.

7.  Sensor meeting the requirements of 1 to 6 **characterized by** the detecting reagents being brought into contact with the electrically non-conductive, permeable sheet by soaking or coating.

8.  Sensor meeting the requirements of 1 to 7 **characterized by** the detecting reagents being glucose oxidase and potassium hexacyanoferrate.

9.  Sensor meeting the requirements of 1 to 8 **characterized by** the electrically conductive, porous sheet consisting of metallic fabric, fleece or membrane.

10. Sensor meeting the requirements of 1 to 9 **characterized by** the electrically conductive, porous sheet consisting of a hydrophilic graphite fibre material, preferably graphite fleece, or a non-conductive hydrophilic fleece with an overlayered conductive metal or graphite foil.

**11.** Sensor meeting the requirements of 1 to 10 **characterized by** the electrically conductive, porous sheet containing other reagents.

**Revendications**

**1.** Capteur ampérométrique, comprenant une électrode de travail et une électrode de référence ainsi qu'au moins un réactif indicateur,
**caractérisé en ce que**
l'électrode de travail et l'électrode de référence sont séparées par une feuille perméable non conductible comportant un réactif indicateur
et **en ce que**
l'électrode de référence est composée d'une feuille poreuse conductible servant au transport capillaire de liquide d'une zone d'échantillonnage à la feuille perméable non conductible.

**2.** Capteur selon la revendication 1, **caractérisé en ce que** la feuille perméable non conductible est composée d'une matière poreuse, par exemple non-tissé, papier, tissu, membrane ou d'une couche de membrane non poreuse susceptible de gonfler.

**3.** Capteur selon les revendications 1 à 2, **caractérisé en ce que** la feuille perméable non conductible est composée de plusieurs couches.

**4.** Capteur selon la revendication 3, **caractérisé en ce que** les différentes couches contiennent différents réactifs indicateurs.

**5.** Capteur selon les revendications 1 à 4, **caractérisé en ce que** la feuille perméable non conductible a une surface de l'ordre de quelques $mm^2$, de préférence de l'ordre de 7 $mm^2$.

**6.** Capteur selon les revendications 1 à 5, **caractérisé en ce que** la taille des pores de la feuille perméable non conductible est de l'ordre de 0,1 à 10 $\mu$m, de préférence de l'ordre de 0,3 à 0,5 $\mu$m.

**7.** Capteur selon les revendications 1 à 6, **caractérisé en ce que** les réactifs indicateurs sont mis en contact avec la feuille perméable non conductible par imprégnation ou par revêtement.

**8.** Capteur selon les revendications 1 à 7, **caractérisé en ce que** les réactifs indicateurs sont la glucose-oxydase et l'hexacyanoferrate de potassium.

**9.** Capteur selon les revendications 1 à 8, **caractérisé en ce que** la feuille poreuse conductible est composée de tissu métallisé, de non-tissé ou de membrane.

**10.** Capteur selon les revendications 1 à 9, **caractérisé en ce que** la feuille poreuse conductible est composée d'une matière en fibre graphite hydrophile, de préférence un non-tissé de graphite, ou d'un non-tissé hydrophile non conductible revêtu d'un film métallique ou de graphite conductible.

**11.** Capteur selon les revendications 1 à 10, **caractérisé en ce que** la feuille poreuse conductible contient d'autres réactifs.

# Fig. 1

(6)

(5)

(3)

(4)

(2)

(1)

(6) (5) (4) (7) (8)

(9)

(3) (2) (1)